(19) **Europäisches Patentamt**

**European Patent Office**

**Office européen des brevets**

(11) **EP 1 329 161 A1**

(12) **EUROPEAN PATENT APPLICATION**
published in accordance with Art. 158(3) EPC

(43) Date of publication:
**23.07.2003 Bulletin 2003/30**

(21) Application number: **01980910.2**

(22) Date of filing: **25.10.2001**

(51) Int Cl.7: **A01N 63/04**, C12N 1/14

(86) International application number:
**PCT/JP01/09394**

(87) International publication number:
**WO 02/035934 (10.05.2002 Gazette 2002/19)**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **27.10.2000 JP 2000329070**
**27.10.2000 JP 2000329071**

(71) Applicant: **Idemitsu Kosan Co., Ltd.**
**Tokyo 100-8321 (JP)**

(72) Inventors:
• **IZU, Susumu**
**Sodegaura-shi, Chiba 299-0293 (JP)**
• **IWAGAMI, Naoko**
**Sodegaura-shi, Chiba 299-0293 (JP)**

(74) Representative:
**Gille Hrabal Struck Neidlein Prop Roos**
**Patentanwälte**
**Brucknerstrasse 20**
**40593 Düsseldorf (DE)**

(54) **AGENTS AND METHOD OF CONTROLLING PLANT FOLIAGE DISEASES**

(57)     The invention provides an agent for preventing and controlling stem and leaf diseases of plants such as typically gray mold, leaf blight and powdery mildew of farm plants, which is not harmful to the natural environment and of which the effect lasts long for protecting farm plants from phytopathogenic fungi that cause such plant diseases, and also provides a method of using the agent for protecting farm plants from such diseases. The agent is for preventing and controlling stem and leaf diseases of plants, and contains cells belonging to the genus *Talaromyces* and antagonistic to fungi that cause stem and leaf diseases of plants; and the method comprises spraying the agent on the stems and leaves of plants to be protected with it.

EP 1 329 161 A1

**Description**

TECHNICAL FIELD

The present invention relates to an agent for preventing and controlling stem and leaf diseases of plants, such as typically gray mold, leaf blight and powdery mildew of farm plants, and relates to a method of using the agent for preventing and controlling stem and leaf diseases of plants such as gray mold, leaf blight and powdery mildew. More precisely, the invention relates to an agent for preventing and controlling plant diseases of gray mold, leaf blight and powdery mildew, which is not harmful to the natural environment and of which the effect for preventing and controlling such plant diseases lasts long, and relates to a method of using the agent for preventing and controlling such plant diseases of gray mold, leaf blight and powdery mildew.

BACKGROUND ART

While being cultivated, crop plants are often damaged by various phytopathogenic fungi. Of such fungi, those to cause gray mold, leaf blight and powdery mildew often grow on plants of strawberry, tomato, eggplant, grape, rose, etc. Once infected with such fungi, the plants will be the source of infection and most plants around them will suffer serious damage. Accordingly, various fungicides are used for preventing and controlling the growth and propagation of fungi that cause stem and leaf diseases of plants such as gray mold, leaf blight and powdery mildew.

In general, most active ingredients of such fungicides are synthetic chemicals, and some of them will be harmful to humans and animals and will be hormone-disturbing chemicals. Used in a farm, in addition, some fungicides take a long period of time before they are completely degraded to be harmless to the natural world, and there is a possibility that the fungicides are often harmful to the natural environment.

In practical application of such fungicides to crop plants, it is often difficult to determine when they are the most effective for exterminating the intended fungi, and, in addition, the effective period of the active ingredients of the fungicides is not so long. For these reasons, the fungicides must be applied to plants frequently and are therefore troublesome.

Further, while the fungicides are continuously used, the targeted phytopathogenic fungi will become resistant to them and the fungicides will be therefore no more effective to the fungi. If so, some other stronger fungicides must be used to kill the fungi.

For producing farm crops not having any negative influences on the natural environment in that situation, plant varieties resistant to phytopathogenic fungi are bred.

However, it is extremely difficult to breed plant varieties that are resistant to phytopathogenic fungi, not detracting from the taste and the quality of the plant crops. In addition, another problem with the breeding is that it takes a long period of time of from 10 to 20 years or so to make the newly-bred variety of plant acclimated in the plant-producing district.

On the other hand, known is a method of preventing and controlling plant diseases by utilizing the antagonistic activity of microorganisms against phytopathogenic fungi. The method will be harmless to the natural environment, but is problematic in that the activity of the microorganisms of preventing and controlling plant diseases does not last long.

Given that situation, it is desired to develop an agent for preventing and controlling stem and leaf diseases of plants such as gray mold, leaf blight and powdery mildew of farm plants, which is not harmful to the natural environment and of which the effect lasts long for protecting farm plants from phytopathogenic fungi that cause such plant diseases, and also to develop a method of using the agent for effectively protecting farm plants from such diseases.

The object of the present invention is to provide an agent for preventing and controlling stem and leaf diseases of plants such as gray mold, leaf blight and powdery mildew of farm plants, which is not harmful to the natural environment and of which the effect lasts long for protecting farm plants from phytopathogenic fungi that cause such plant diseases, and also to provide a method of using the agent for effectively protecting farm plants from such diseases.

DISCLOSURE OF THE INVENTION

We, the present inventors have assiduously studied so as to solve the problems noted above and, as a result, have found that some fungi belonging to the genus *Talaromyces* are antagonistic to the fungi that cause stem and leaf diseases of plants such as gray mold, leaf blight and powdery mildew. On the basis of this finding, we have completed the present invention.

Specifically, the invention is summarized as follows:

(1) An agent for preventing and controlling stem and leaf diseases of plants, which contains cells belonging to the genus *Talaromyces* and antagonistic to fungi that cause stem and leaf diseases of plants.

(2) An agent for preventing and controlling stem and leaf diseases of plants, which comprises from 1 to 90 % by mass of cells belonging to the genus *Talaromyces* and antagonistic to fungi that cause stem and leaf diseases of plants, from 1 to 15 % by mass of a surfactant, and from 0 to 98 % by mass (including 0) of an extender.

(3) An agent for preventing and controlling stem and leaf diseases of plants, which comprises from 2 to 46 % by mass of cells belonging to the genus *Talaromyces* and antagonistic to fungi that cause stem and leaf diseases of plants, from 2 to 15 % by mass of a surfactant, and from 39 to 96 % by mass of an extender.

(4) The agent for preventing and controlling stem and leaf diseases of plants of any of above (1) to (3), wherein the cells belonging to the genus *Talaromyces* are those of *Talaromyces flavus*.

(5) The agent for preventing and controlling stem and leaf diseases of plants of any of above (1) to (4), wherein the cells belonging to the genus *Talaromyces* are those of *Talaromyces flavus* Y-9401.

(6) The agent for preventing and controlling stem and leaf diseases of plants of any of above (1) to (5), wherein the cells belonging to the genus Talaromyces are cultured cells of from $1 \times 10^6$ to $1 \times 10^{12}$/g in terms of the colony-forming units, or ground powder or spores thereof.

(7) The agent for preventing and controlling stem and leaf diseases of plants of above (2) or (3), wherein the surfactant is one or more selected from fatty acid soaps, alkylether-carboxylic acids, N-acylamino acids, salts of alkylbenzenesulfonic acids, salts of alkylnaphthalenesulfonic acids, salts of $\alpha$-olefinsulfonic acids, salts of dialkylsulfosuccinate esters, salts of higher alcohol sulfate esters, salts of alkylether-sulfuric acids, salts of polyoxyethylene-alkylphenylether-sulfuric acids, salts of fatty acid alkylolamide sulfate esters, salts of alkylether phosphate esters, salts of alkyl phosphate esters, salts of aliphatic amines, aliphatic quaternary ammonium salts, benzalkonium salts, benzetonium chloride, pyridinium salts, imidazolinium salts, polyoxyethylene alkyl ethers, polyoxyethylene alkylphenyl ethers, polyoxyethylene-polyoxypropylene block polymers, polyoxyethylene-polyoxypropylene alkyl ethers, polyoxyethylene glycerin fatty acid esters, polyoxyethylene castor oil, polyoxyethylene sorbitan fatty acid esters, polyoxyethylene sorbitol fatty acid esters, polyethylene glycol fatty acid esters, fatty acid monoglycerides, polyglycerin fatty acid esters, sorbitan fatty acid esters, fatty acid alkanolamides, polyoxyethylene fatty acid amides, polyoxyethylene-alkylamines, alkylamine oxides, carboxybetaines, salts of aminocarboxylic acids, and imidazolinium betaines.

(8) The agent for preventing and controlling stem and leaf diseases of plants of any of above (2) or (3), wherein the extender is one or more selected from kaolin clay, pyrophyllite clay, bentonite, montmorillonite, diatomaceous earth, synthetic silicon oxide hydrate, acid clay, talc, clay, ceramic, quartz, sericite, vermiculite, pearlite, Ohya stone, anthrax, limestone, coal ash, zeolite and attapulgite.

(9) A method for preventing and controlling stem and leaf diseases of plants, which comprises spraying the stems and leaves of plants with an agent for preventing and controlling stem and leaf diseases of plants and in which the agent contains cells belonging to the genus *Talaromyces* and antagonistic to fungi that cause stem and leaf diseases of plants.

(10) A method for preventing and controlling stem and leaf diseases of plants, which comprises, spraying the stems and leaves of plants with a 50-fold to 3000-fold wet dilution of an agent for preventing and controlling stem and leaf diseases of plants and in which the agent comprises from 1 to 90 % by mass of cells belonging to the genus *Talaromyces* and antagonistic to fungi that cause stem and leaf diseases of plants, from 1 to 15 % by mass of a surfactant, and from 0 to 98 % by mass of an extender.

(11) A method for preventing and controlling stem and leaf diseases of plants, which comprises spraying the stems and leaves of plants with a 50-fold to 3000-fold wet dilution of an agent for preventing and controlling stem and leaf diseases of plants and in which the agent comprises from 2 to 46 % by mass of cells belonging to the genus *Talaromyces* and antagonistic to fungi that cause stem and leaf diseases of plants, from 2 to 15 % by mass of a surfactant, and from 39 to 96 % by mass of an extender.

(12) The method for preventing and controlling stem and leaf diseases of plants of any of above (9) to (11), wherein the cells belonging to the genus *Talaromyces* are those of *Talaromyces flavus.*

(13) The method for preventing and controlling stem and leaf diseases of plants of any of above (9) to (12), wherein the cells belonging to the genus *Talaromyces* are those of *Talaromyces flavus* Y-9401.

(14) The method for preventing and controlling stem and leaf diseases of plants of any of above (9) to (13), wherein the cells belonging to the genus *Talaromyces* are cultured cells of from $1 \times 10^6$ to $1 \times 10^{12}$/g in terms of the colony-forming units, or ground powder or spores thereof.

(15) The method for preventing and controlling stem and leaf diseases of plants of above (10) or (11), wherein the surfactant is one or more selected from fatty acid soaps, alkylether-carboxylic acids, N-acylamino acids, salts of alkylbenzenesulfonic acids, salts of alkylnaphthalenesulfonic acids, salts of $\alpha$-olefinsulfonic acids, salts of dialkylsulfosuccinate esters, salts of higher alcohol sulfate esters, salts of alkylether-sulfuric acids, salts of polyoxyethylene-alkylphenylether-sulfuric acids, salts of fatty acid alkylolamide sulfate esters, salts of alkylether phosphate esters, salts of alkyl phosphate esters, salts of aliphatic amines, aliphatic quaternary ammonium salts, benzalkonium salts, benzetonium chloride, pyridinium salts, imidazolinium salts, polyoxyethylene alkyl ethers, polyoxyethylene alkylphenyl ethers, polyoxyethylene-polyoxypropylene block polymers, polyoxyethylene-polyoxypropylene alkyl ethers, polyoxyethylene glycerin fatty acid esters, polyoxyethylene castor oil, polyoxyethylene sorbitan fatty acid esters, polyoxyethylene sorbitol fatty acid esters, polyethylene glycol fatty acid esters, fatty acid monoglycerides, polyglycerin fatty acid esters, sorbitan fatty acid esters, fatty acid alkanolamides, polyoxyethylene fatty acid amides, polyoxyethylene-alkylamines, alkylamine oxides, carboxybetaines, salts of aminocarboxylic acids, and imidazolinium betaines.

(16) The method for preventing and controlling stem and leaf diseases of plants of any of above (10) or (11), wherein the extender is one or more selected from kaolin clay, pyrophyllite clay, bentonite, montmorillonite, diatomaceous earth, synthetic silicon oxide hydrate, acid clay, talc, clay, ceramic, quartz, sericite, vermiculite, pearlite, Ohya stone, anthrax, limestone, coal ash, zeolite and attapulgite.

(17) The agent for preventing and controlling stem and leaf diseases of plants of any of above (1) to (8), which is for gray mold, leaf blight or powdery mildew.

(18) The method for preventing and controlling stem and leaf diseases of plants of any of above (9) to (16), which is for gray mold, leaf blight or powdery mildew.

## BEST MODES OF CARRYING OUT THE INVENTION

Embodiments of the invention are described below.

The invention is an agent for preventing and controlling stem and leaf diseases of plants such as gray mold, leaf blight and powdery mildew, and the agent contains cells belonging to the genus *Talaromyces* and antagonistic to fungi that cause stem and leaf diseases of plants such as typically gray mold, leaf blight and powdery mildew of farm plants; and the invention is also a method of using the agent for preventing and controlling such stem and leaf diseases of plants. Of cells that belong to the genus *Talaromyces*, those of *Talaromyces flavus, Talaromyces bacillisporus, Talaromyces helicum, Talaromyces luteus* or *Talaromyces rutundus* are antagonistic to any or all fungi that cause plant diseases of gray mold, leaf blight or powdery mildew. Above all, the cells of *Talaromyces flavus* are the most antagonistic to the phytopathogenic fungi and are favorable to the invention. Of the cells of *Talaromyces flavus*, those of *Talaromyces flavus* Y-9401 are especially strongly antagonistic to any or all fungi that cause plant diseases of gray mold, leaf blight or powdery mildew, and are therefore more favorable to the invention. The effect of the cells of *Talaromyces* to antagonize the fungi that cause gray mold or leaf blight may be confirmed, for example, as follows: Both the cells of *Talaromyces* and the cells to cause gray mold or leaf blight are put on one and the same plate, and then they are incubated at 25 to 37°C for 3 to 7 days while the cells of the two groups are made to face each other on the plate on which they are incubated. After thus incubated, the growing condition of the phytopathogenic cells retarded by the cells of *Talaromyces* is observed.

On the other hand, the effect of the cells of *Talaromyces* to antagonize the fungi that cause powdery mildew may be confirmed, for example, as follows: Both the cells of *Talaromyces* and the cells to cause powdery mildew are put a strawberry leaf, and then they are incubated at 15 to 20°C for 1 to 3 days while the cells of the two groups are made to face each other on the leaf on which they are incubated. After thus incubated, the growing condition of the phytopathogenic cells retarded by the cells of *Talaromyces* is observed.

Cells of *Talaromyces* may be incubated and grown in the same manner as that for incubating and growing ordinary mold fungi. For culturing the cells, employable is a method of liquid culture in a reciprocating shaker fermenter or a jar fermenter, or a method of solid culture. In the invention, preferably used are spores cultured in a mode of solid culture as the yield thereof is high. For the medium component to be used in culturing the cells of *Talaromyces,* for example, usable is a potato-dextrose medium or a Sabouraud's medium. For solid culture of the cells, for example, preferred is a solid medium that is prepared by infiltrating cereals such as rice, wheat and corn as well as other cereals-derived solid components such as bran, and other nutrient sources such as saccharides and nitrogen sources into a porous carrier of, for example, clay minerals. It is desirable that the cells are cultured under aerobic conditions, for example, by aerial stirring or shaking incubation. Preferably, the culture temperature falls between 20 and 37°C, and the culture period falls between 3 and 60 days, more preferably between 3 and 14 days, or between 30 and 60 days depending on the culture conditions employed.

The cultures thus obtained may be used for the active ingredients of the agent for preventing and controlling plant diseases of gray mold, leaf blight or powdery mildew. Preferably, the concentration of the cells in the agent falls between $1 \times 10^6$ and $1 \times 10^{12}$/g, more preferably between $1 \times 10^7$ and $1 \times 10^{12}$/g in terms of the colony-forming units. Thus obtained, the cultures may be directly used for the active ingredients of the agent for preventing and controlling plant diseases of gray mold, leaf blight or powdery mildew, but if desired, they may be ground or cut into pieces, or may be centrifuged to isolate the cultured cells from the cultures. Further if desired, they may be dried before use. Still if desired, spores may be collected from the cultures, then dried, ground and classified before use.

The agent for preventing and controlling plant diseases of gray mold, leaf blight or powdery mildew, which contains, as the active ingredient, the cells of *Talaromyces* prepared in the manner as above is preferably so formulated that (1) it comprises from 1 to 90 % by dry mass of the cells of *Talaromyces*, from 1 to 15 % by mass of a surfactant, and from 0 to 98 % by mass of an extender; or (2) it comprises from 2 to 46 % by dry mass of the cells of *Talaromyces*, from 2 to 15 % by mass of a surfactant, and from 39 to 96 % by mass of an extender, as they are favorable for spraying on the stems and leaves of plants. In the composition (1) or (2) for powdery mildew, the amount of the extender preferably falls between 65 and 90 % by mass. Specifically, if the amount of the cells of *Talaromyces* in the agent is smaller than 1 % by mass, the agent is often ineffective for preventing and controlling the targeted plant diseases of gray mold, leaf blight or powdery mildew; but if it is larger than 90 % by mass, a wettable powder of the agent suitable to spraying on plants will be difficult to prepare. If the amount of the surfactant in the agent is smaller than 1 % by mass, the cells in the agent could not well disperse in water when the agent is diluted with water to prepare an aqueous spray of the agent; but even if the amount of the surfactant is increased over 15 % by mass, the cell dispersibility in water will be no more improved. If the amount of the extender in the agent is larger than 98 % by mass, the content of the active ingredient in the agent will lower and, if so, the agent will be ineffective for preventing and controlling the targeted plant diseases of gray mold, leaf blight or powdery mildew.

The surfactant to be in the agent of the invention must have the ability to improve the dispersibility of the cells of *Talaromyces* in water and must not have any negative influence on the cells of *Talaromyces*. Surfactants that have the properties and are suitable to the agent of the invention are, for example, fatty acid soaps, alkylether-carboxylic acids, N-acylamino acids, salts of alkylbenzenesulfonic acids, salts of alkylnaphthalenesulfonic acids, salts of $\alpha$-olefinsulfonic acids, salts of dialkylsulfosuccinate esters, salts of higher alcohol sulfate esters, salts of alkylether-sulfuric acids, salts of polyoxyethylene-alkylphenylether-sulfuric acids, salts of fatty acid alkylolamide sulfate esters, salts of alkylether phosphate esters, salts of alkyl phosphate esters, salts of aliphatic amines, aliphatic quaternary ammonium salts, benzalkonium salts, benzetonium chloride, pyridinium salts, imidazolinium salts, polyoxyethylene alkyl ethers, polyoxyethylene alkylphenyl ethers, polyoxyethylene-polyoxypropylene block polymers, polyoxyethylene-polyoxypropylene alkyl ethers, polyoxyethylene glycerin fatty acid esters, polyoxyethylene castor oil, polyoxyethylene sorbitan fatty acid esters, polyoxyethylene sorbitol fatty acid esters, polyethylene glycol fatty acid esters, fatty acid monoglycerides, polyglycerin fatty acid esters, sorbitan fatty acid esters, fatty acid alkanolamides, polyoxyethylene fatty acid amides, polyoxyethylene-alkylamines, alkylamine oxides, carboxybetaines, salts of aminocarboxylic acids, and imidazolinium betaines. One or more of these surfactants may be in the agent of the invention.

of those surfactants, especially preferred are salts of alkylbenzenesulfonic acids, salts of alkylnaphthalenesulfonic acids, and salts of $\alpha$-olefinsulfonic acids, as their ability to assist cell dispersion in water is good.

The extender to be used herein is preferably one having the ability to form a well-handlable homogeneous mixture when mixed with the cells of *Talaromyces*. The extender of the type includes, for example, kaolin clay, pyrophyllite clay, bentonite, montmorillonite, diatomaceous earth, synthetic silicon oxide hydrate, acid clay, talc, clay, ceramic, quartz, sericite, vermiculite, pearlite, Ohya stone, anthrax, limestone, coal ash, zeolite and attapulgite. One or more of these extenders may be in the agent of the invention.

When the agent of the invention, which is for preventing and controlling stem and leaf diseases of plants such as gray mold, leaf blight or powdery mildew and which contains, as the active ingredient, the cells of *Talaromyces* is used for preventing and controlling the targeted stem and leaf diseases of plants such as gray mold, leaf blight or powdery mildew of crop plants, for example, the agent is preferably sprayed on the stems and leaves of crop plants to be protected from the diseases. Preferably, the spray is prepared by diluting the agent that comprises the cells of *Talaromyces*, the surfactant and the extender, with water into a 50-fold to 3000-fold wet dilution thereof. This is because if the dilution ratio is less than 50 times, the cell dispersibility in water will be low; but if over than 3000 times, the effective cell concentration in the resulting dilution will be too low and the dilution will be ineffective for preventing and controlling the targeted plant cells. More preferably, the dilution ration with water falls between 500 and 2000 times.

Regarding the time when the agent is sprayed on the stems and leaves of crop plants, the agent may be sprayed thereon anytime of the stage of young seedlings, the stage of temporary plantation, the stage of fixed plantation or the stage of crop harvesting for vegetables such as strawberry, tomato and eggplant and for rose. For grape, it is desirable that the agent is sprayed on the stems and leaves of the plants before or after the stage of blooming. Regarding the frequency of spraying the agent, the duration period of the antagonistic effect of the cells of *Talaromyces* against the fungi that cause stem and leaf diseases of plants such as gray mold, leaf blight or powdery mildew may be 2 months or more. Therefore, the agent spraying frequency may be once per 1 to 3 months for satisfactorily protecting the plants from the plant diseases such as gray mold, leaf blight or powdery mildew.

The invention is described more concretely with reference to the following Examples, which, however, are not intended to restrict the scope of the invention.

[Example 1]

(1) Culture of *Talaromyces flavus* Y-9401:

Bran was used for the culture medium. Seed cells of *Talaromyces flavus* Y-9401 (FERM P-15816) were planted in this, and cultured at 30°C for 10 days in a mode of solid culture.
After thus cultured, the resulting culture was dried and sieved to obtain a ground powder of *Talaromyces flavus* Y-9401 cells.

(2) Preparation of agent for preventing and controlling gray mold, leaf blight or powdery mildew:

5 g of the ground powder of *Talaromyces flavus* Y-9401 cells that had been prepared in (1), 10 g of a surfactant alkylnaphthalene sulfonate, and 85 g of an extender, clay mineral (K Clay by Shokohzan Mining Factory) were mixed to prepare an agent for preventing and controlling gray mold, leaf blight or powdery mildew.

(3) Evaluation of the agent for strawberry gray mold:

In October of last year, strawberry seedlings (variety: Nyoho) were planted in a polyvinyl film greenhouse. Six months after the plantation, the agent prepared in (2) was sprayed on the strawberry plants to evaluate its effect for preventing and controlling gray mold. The planting condition is as follows: The distance between the neighboring ridges is 115 cm; the distance between the neighboring seedlings is 20 cm; 2 lines of seedlings were planted in one ridge; and the planting density is 870 seedlings/are.
The agent prepared in (2) as a wettable agent was diluted with water into a 1000-fold dilution and before sprayed on the plants, a spreader (Kao's Approach BI) was added thereto in an amount to give the same concentration as the agent. The resulting preparation was used as a spray preparation for strawberry. The amount of the spray preparation applied to the strawberry plants was 150 liters/10 ares. A sprayer was used for spraying the preparation on the stems and leaves of the strawberry plants. For 16 hours after the spraying, the greenhouse was airtightly closed. The spraying frequency for protecting the strawberry plants from gray mold is as follows: The second spraying is on the day 7th after the first spraying, the third spraying is on the day 7th after the second spraying, and the fourth spraying is on the day 6th after the third spraying.
For comparison, a commercially-available fungicide for gray mold (Nippon Agricultural Chemicals' Polyoxin AL wettable powder) was diluted with water into a 1000-fold dilution, and for protecting from gray mold this was sprayed on strawberry plants that were grown in the same manner as above but in a different polyvinyl film greenhouse.
Still in a different polyvinyl film greenhouse, strawberry plants were grown in the same manner as above, but were not sprayed with any preparation for preventing and controlling gray mold (this is a control group).
Having been thus grown in every greenhouse, 15 strawberry plants were sampled from the center of each greenhouse and checked for gray mold appearing in stems and leaves and that appearing in peduncles. According to the following equations, the morbidity of diseased strawberry plants, and the morbidity of diseased strawberry fruits were calculated.

$$\text{Morbidity (\%) of diseased plants} = [(\text{number of diseased plants})/(\text{number of all plants checked})] \times 100$$

$$\text{Morbidity (\%) of diseased fruits} = [(\text{number of diseased fruits})/(\text{number of all fruits checked})] \times 100$$

In addition, the morbidity of diseased plants in the control group (with no spray) was calculated, and the effect of the preparation tried herein for protecting strawberry plants from gray mold was calculated according to the following equation:

$$\text{Effectiveness (\%) against gray mold} = \{[(\text{morbidity of diseased plants in control section with no spray}) - (\text{morbidity of diseased plants in test section with spray})]/(\text{ morbidity of diseased plants in control section with no spray})\} \times 100$$

The test data of the preparation for protecting strawberry plants from gray mold are shown in Table 1.

Table 1

| | Morbidity (%) of Diseased Plants | Effectiveness (%) against Gray Mold | Morbidity (%) of Diseased Fruits | Effectiveness (%) against Gray Mold |
|---|---|---|---|---|
| Example 1 | 20.0 | 72.7 | 4.5 | 74.0 |
| Comparative Example 1 | 23.0 | 68.6 | 6.0 | 65.3 |
| (Control with no spray) | 73.3 | - | 17.3 | - |

**[Example 2]**

In October of last year, tomato seedlings (variety: House Momotaro) were planted in a glass greenhouse. Six months after the plantation, the plants were found to have been infected with gray mold. The agent that had been prepared in (2) of Example 1 was tried for the plants to evaluate its effect. The planting condition is as follows: The distance between the neighboring ridges is 200 cm; the distance between the neighboring seedlings is 50 cm; 2 lines of seedlings were planted in one ridge; and the distance between the 2 lines of seedlings is 80 cm.

The agent prepared in (2) of Example 1 as a wettable agent was diluted with water into a 1000-fold dilution, and before sprayed on the plants, a spreader (Nippon Agricultural Chemicals' Mairino) was added thereto in an amount to give the same concentration as the agent. The resulting preparation was used as a spray preparation for tomato. Before the spraying test, the diseased fruits were pinched off. The amount of the spray preparation applied to the tomato plants was 350 liters/10 ares. A sprayer was used for spraying the preparation on the stems and leaves of the tomato plants. For 16 hours after the spraying, the greenhouse was airtightly closed. The spraying frequency for protecting the tomato plants from gray mold is as follows: The second spraying is on the day 7th after the first spraying, and the third spraying is on the day 7th after the second spraying.

For comparison, a commercially-available fungicide for gray mold (Nissan Chemical's Robural wettable powder) was diluted with water into a 1000-fold dilution, and for protecting from gray mold this was sprayed on tomato plants that were grown in the same manner as above but in a different glass greenhouse.

Still in a different glass greenhouse, tomato plants were grown in the same manner as above, but were not sprayed with any preparation for preventing and controlling gray mold (this is a control group).

Having been thus grown in every greenhouse, 20 tomato plants were sampled from the center of each greenhouse and checked for gray mold appearing in fruits. According to the following equation, the morbidity of diseased tomato fruits was calculated.

$$\text{Morbidity (\%) of diseased fruits} = [(\text{number of diseased fruits})/(\text{number of all fruits checked})] \times 100$$

The test data of the preparation for protecting tomato plants from gray mold are shown in Table 2.

Table 2

| | Morbidity (%) of Diseased Fruits | Effectiveness (%) against Gray Mold |
|---|---|---|
| Example 2 | 1.5 | 75.4 |
| Comparative Example 2 | 2.5 | 59.0 |
| (Control with no spray) | 6.1 | - |

[Example 3]

The agent that had been prepared in (2) of Example 1 was diluted with water into a 1000-fold dilution, and this was sprayed on the ears of 13-year-old grape trees (variety: Kyoho) that are cultivated in a greenhouse. This is for protecting the grape trees from gray mold. The spraying was effected three times as follows: The first and second spraying is on the day 23rd and day 11th before the estimated ear blooming date, and the third spraying is on the day 10th after the ear blooming.

The amount of the spray applied to the grape trees was 50 liters/100 m$^2$ in every spraying operation. For the spraying, used was a power-driven sprayer.

For comparison, a commercially-available fungicide (Nissan Chemical's Robural wettable powder) was diluted with water into a 1500-fold dilution, and, for protecting from gray mold this was sprayed in the same manner as above on the ears of grape trees that are cultivated in a different greenhouse.

For control, grape trees in a still different greenhouse are kept cultivated as they are, with no agent for gray mold sprayed thereon (this is a control group).

Having been thus grown in every greenhouse, 200 grape ears were sampled from the center of each greenhouse and checked for gray mold appearing therein. According to the following equation, the morbidity of diseased grape ears was calculated.

Morbidity (%) of diseased ears = [(number of diseased ears)/(number of all ears checked)]×100

The test data of the preparation for protecting grapes from gray mold are shown in Table 3.

Table 3

| | Morbidity (%) of Diseased Ears | Effectiveness (%) against Gray Mold |
|---|---|---|
| Examples | 5.0 | 80.0 |
| Comparative Example 3 | 10.0 | 60.0 |
| (Control with no spray) | 25.0 | |

[Example 4]

In October of last year, strawberry seedlings (variety: Nyoho) were planted in a polyvinyl film greenhouse. Six months after the plantation, the plants were sprayed with the agent for gray mold that had been prepared in (2) of Example 1, in the same manner as in (3) in Example 1. This is for evaluating the agent for protecting the strawberry plants from leaf blight.

For comparison, a commercially-available fungicide for gray mold (Sankyo Pharmaceutical's Bercute wettable powder) was diluted with water into a 1000-fold dilution, and this was tried.

Having been thus grown in every greenhouse, 15 strawberry plants were sampled from the center of each greenhouse and checked for leaf blight. According to the following equation, the morbidity of diseased strawberry plants were calculated.

Morbidity (%) of diseased plants = [(number of diseased leaves)/(number of all leaves checked)] × 100

In addition, the morbidity of diseased plants in a control group (with no spray) was calculated in the same manner as above, and the effect of the preparation tried herein for protecting strawberry plants from leaf blight was calculated according to the following equation:

Effectiveness (%) against leaf blight = {[(morbidity of diseased leaves in control section with no

spray) - (morbidity of diseased leaves in test section with

spray)]/( morbidity of diseased leaves in control section with no spray)} × 100

The test data of the preparation for protecting strawberry plants from leaf blight are shown in Table 4.

Table 4

| | Morbidity (%) of Diseased Leaves | Effectiveness (%) against Leaf Blight |
|---|---|---|
| Example 4 | 4.6 | 88.2 |
| Comparative Example 4 | 10.5 | 73.1 |

Table 4 (continued)

|  | Morbidity (%) of Diseased Leaves | Effectiveness (%) against Leaf Blight |
|---|---|---|
| (Control with no spray) | 39.0 | - |

[Example 5]

(1) Evaluation of the agent for strawberry powdery mildew:

In September of last year, strawberry seedlings (variety: Toyonoka) were planted in a polyvinyl film greenhouse. Eight months after the plantation, the agent prepared in (2) of Example 1 was sprayed on the strawberry plants to evaluate its effect for preventing and controlling powdery mildew. The planting condition is as follows: The distance between the neighboring ridges is 100 cm; the distance between the neighboring seedlings is 25 cm; and the planting frequency is 10 plants/section. In the polyvinyl film greenhouse, strawberry plants infected with powdery mildew were put, and the aerial infection of powdery mildew to the other plants in the greenhouse was promoted.

The agent prepared in (2) of Example 1 as a wettable agent was diluted with water into a 1000-fold dilution and used as a spray preparation for the strawberry. After it was confirmed that the strawberry plants in the greenhouse were infected with powdery mildew, the dilution was sprayed on the plants. The amount of the spray preparation applied to the strawberry plants was 200 liters/10 ares. A sprayer was used for spraying the preparation on the stems and leaves of the strawberry plants. The spraying frequency is as follows: The second spraying is on the day 7th after the first spraying, and the third spraying is on the day 7th after the second spraying. This is for exterminating the powdery mildew fungi from the strawberry plants.

For comparison, a commercially-available fungicide for powdery mildew (Nippon Soda's Trifumin wettable powder) was diluted with water into a 3000-fold dilution, and for exterminating the powdery mildew fungi this was sprayed on strawberry plants that were grown in the same manner as above but in a different polyvinyl film greenhouse.

Still in a different polyvinyl film greenhouse, strawberry plants were grown in the same manner as above, but were not sprayed with any preparation for preventing and controlling powdery mildew (this is a control group).

Having been thus grown in every greenhouse, all the strawberry plants were checked three times, (1) on the day of the second spraying, (2) on the day of the third spraying, and (3) on the day 7th after the third spraying, for infection with powdery mildew. According to the following equations, the morbidity of diseased leaves, and the degree of disease were calculated.

$$\text{Morbidity (\%) of diseased leaves} = [(\text{number of diseased leaves})/(\text{number of all leaves checked})] \times 100$$

$$\text{Degree of disease} = \{[\Sigma(\text{number of leaves diseased in different degrees} \times \text{index})]/[(\text{number of all leaves checked}) \times 4]\} \times 100$$

The index in the equation to give the degree of disease is as follows: 0 is given to plants with no disease; 0.5 is given to plants diseased only a little; 1 is given to plants of which the diseased area is less than 5 %; 2 is given to plants of which the diseased area is from 5 % to less than 25 %; 3 is given to plants of which the diseased area is from 25 % to less than 50 %; and 4 is given to plants of which the diseased area is 50 % or more.

In addition, the morbidity of diseased leaves in the control group (with no spray) was calculated, and the effect of the preparation tried herein for protecting strawberry plants from powdery mildew was calculated according to the following equation:

$$\text{Effectiveness (\%) against powdery mildew} = \{[(\text{morbidity of diseased leaves in control section with no spray}) - (\text{morbidity of diseased leaves in test section with spray})]/(\text{morbidity of diseased leaves in control section with no spray})\} \times 100$$

The test data of the preparation for protecting strawberry plants from powdery mildew are shown in Table 5.

Table 5

| | Morbidity (%) of diseased leaves | | | Degree of Disease | | | Effectiveness against powdery mildew |
|---|---|---|---|---|---|---|---|
| Test Date[*)] | (1) | (2) | (3) | (1) | (2) | (3) | - |
| Example 5 | 6.7 | 11.3 | 14.9 | 1.8 | 2.9 | 3.8 | 91.9 |
| Comparative Example 5 | 16.4 | 22.0 | 40.9 | 5.1 | 6.9 | 20.3 | 57.1 |
| (Control with no spray) | 22.2 | 42.2 | 67.8 | 9.6 | 17.1 | 47.2 | - |

*)

(1): the day of the second spraying,

(2): the day of the third spraying,

(3): the day 7th after the third spraying.

[Example 6]

In a polyvinyl film greenhouse, two-leaves-stage cucumber seedlings (variety: Encore 8) were planted. Before planted therein, those diseased with powdery mildew were brushed to remove the spores of the fungi from their leaves. The agent that had been prepared in (2) of Example 1 was sprayed on these plants to evaluate its effect for protecting the plants from powdery mildew. The planting condition is as follows: The distance between the neighboring ridges is 220 cm; the distance between the neighboring seedlings is 50 cm; and the planting frequency is 12 plants/section. If desired, a liquid fertilizer was applied to the plants.
The agent prepared in (2) of Example 1 as a wettable agent was diluted with water into a 1000-fold dilution and used as a spray preparation for the cucumber seedlings. The amount of the spray preparation applied to the cucumber plants was 130 to 280 liters/10 ares, and it was increased with the growth of the plants. A sprayer was used for spraying the preparation on the stems and leaves of the plants.
The spraying frequency is as follows: The first spraying is on the day 12th (4-leaves-stage) after the plantation, the second spraying is on the day 9th after the first spraying, the third spraying is on the day 8th after the second spraying, and the fourth spraying is on the day 7th after the third spraying. This is for protecting the cucumber plants from powdery mildew.
For comparison, a commercially-available fungicide for powdery mildew (Shionogi Pharmaceutical's Summermachine 97 wettable powder) was diluted with water into a 200-fold dilution, and for protecting from powdery mildew this was sprayed on cucumber plants that were grown in the same manner as above but in a different polyvinyl film greenhouse.
Still in a different polyvinyl film greenhouse, cucumber plants were grown in the same manner as above, but were not sprayed with any preparation for preventing and controlling powdery mildew (this is a control group).
Having been thus grown in every greenhouse, all the cucumber plants were checked three times, (1) on the day 6th after the final spraying, (2) on the day 13th after the final spraying, and (3) on the day 20th after the final spraying, for infection with powdery mildew. Concretely, ten leaves above the first four leaves of every plant were checked as to how and to what degree they were infected with powdery mildew. According to the following equations, the morbidity of diseased leaves, the degree of disease, and the effectiveness against powdery mildew were calculated. For the degree of disease, the index is the same as in Example 5.

Morbidity (%) of diseased leaves = [(number of diseased leaves)/(number of all leaves checked)]

$\times$ 100

Degree of disease

= {[$\Sigma$(number of leaves diseased in different degrees $\times$

index)]/[(number of all leaves checked) $\times$ 4]} $\times$ 100

Effectiveness (%) against powdery mildew = {[(morbidity of diseased leaves in control section with no

spray) - (morbidity of diseased leaves in test section with

spray)]/( morbidity of diseased leaves in control section with

no spray)} $\times$ 100

The test data of the preparation for protecting cucumber plants from powdery mildew are shown in Table 6.

Table 6

| | Morbidity (%) of diseased leaves | | | Degree of Disease | | | Effectiveness against powdery mildew |
|---|---|---|---|---|---|---|---|
| Test Date[*)] | (1) | (2) | (3) | (1) | (2) | (3) | - |
| Example 6 | 0.0 | 0.0 | 33.1 | 0.0 | 0.0 | 6.4 | 91.7 |
| Comparative Example 6 | 16.4 | 34.7 | 57.2 | 2.1 | 5.0 | 10.7 | 86.0 |
| (Control with no spray) | 100 | 100 | 100 | 35.6 | 65.8 | 76.2 | - |
| *) <br> (1): the day 6th after the final spraying, <br> (2): the day 13th after the final spraying, <br> (3): the day 20th after the final spraying. | | | | | | | |

[Example 7]

The agent that had been prepared in (2) of Example 1 was diluted with water into a 1000-fold dilution, and this was sprayed on the ears of 13-year-old grape trees (variety: Kyoho) that are cultivated in a greenhouse. This is for protecting the grape trees from powdery mildew. In the greenhouse, grapes infected with powdery mildew were put, and the aerial infection of powdery mildew to the grape trees in the greenhouse was promoted. The spraying was effected three times as follows: The first and second spraying is on the day 23rd and day 11th before the estimated ear blooming date, and the third spraying is on the day 10th after the ear blooming.
The amount of the spray applied to the grape trees was 50 liters/100 m$^2$ in every spraying operation. For the spraying, used was a power-driven sprayer.
For comparison, a commercially-available fungicide for powdery mildew (Hokko Chemical's Topjin M) was diluted with water into a 1000-fold dilution, and for protecting from powdery mildew this was sprayed in the same manner as above on the ears of grape trees that are cultivated in a different greenhouse.
For control, grape trees in a still different greenhouse are kept cultivated as they are, with no agent for powdery mildew sprayed thereon (this is a control group).
Having been thus grown in every greenhouse, 200 grape ears were sampled from the center of each greenhouse and checked for powdery mildew appearing therein. According to the following equation, the morbidity of diseased grape ears was calculated.

Morbidity (%) of diseased ears = [(number of diseased ears)/(number of all ears checked)]$\times$100

In addition, in the same manner as in Example 5, the effectiveness of the preparation against grape powdery mildew was calculated. The test data of the preparation for protecting grapes from powdery mildew are shown in Table 7.

Table 7

|  | Morbidity (%) of Diseased Ears | Effectiveness (%) against powdery mildew |
|---|---|---|
| Example 7 | 3.2 | 79.5 |
| Comparative Example 7 | 5.1 | 67.3 |
| (Control with no spray) | 15.6 | - |

[Example 8]

In a polyvinyl film greenhouse with no heater for it, five 14-years-old rose plants (variety: Sonia) were planted in the soil bed. The planting condition is as follows: The distance between the neighboring beds is 50 cm; the distance between the neighboring plants is 25 cm; and the plants are in one line. The agent that had been prepared in (2) of Example 1 was diluted with water into a 1000-fold dilution, and this was sprayed on the rose plants for protecting the plants from powdery mildew. In the greenhouse, roses infected with powdery mildew were put, and the aerial infection of powdery mildew to the other rose plants in the greenhouse was promoted. Before the agent dilution was sprayed on the rose plants, it was confirmed that new shoots are growing in all the rose plants and that the leaves of the rose plants are infected with powdery mildew. The spraying was effected two times at an interval of 6 days. The amount of the spray applied to the rose plants was 400 liters/10 ares in every spraying operation.
For comparison, a commercially-available fungicide for powdery mildew (Hokko Chemical's Topjin M) was diluted with water into a 1000-fold dilution, and for protecting from powdery mildew this was sprayed in the same manner as above on rose plants that are cultivated in a different greenhouse and have growing new shoots.
For control, new shoots of rose plants in a still different greenhouse are kept cultivated as they are, with no agent for powdery mildew sprayed thereon (this is a control group).
Having been thus grown in every greenhouse, the rose plants were checked for powdery mildew appearing in the growing new shoots thereof on the day 8 after the final spraying. The degree of disease and the effectiveness of the preparation against powdery mildew were calculated in the same manner as in Example 5. The index for the degree of disease is as follows: 0 is given to plants with no disease; 1 is given to plants of which the diseased area is less than 25 %; 2 is given to plants of which the diseased area is from 25 % to less than 50 %; 3 is given to plants of which the diseased area is from 50 % to less than 75 %; and 4 is given to plants of which the diseased area is 75 % or more. The test data of rose are given in Table 8.

Table 8

|  | Degree of Disease | Effectiveness against powdery mildew |
|---|---|---|
| Example 8 | 5.0 | 74.0 |
| Comparative Example 8 | 7.5 | 60.9 |
| (Control with no spray) | 19.2 | - |

INDUSTRIAL APPLICABILITY

The invention provides an agent for preventing and controlling stem and leaf diseases of plants such as gray mold, leaf blight and powdery mildew of farm plants, which is not harmful to the natural environment and of which the effect lasts long for protecting farm plants from phytopathogenic fungi that cause such plant diseases, and also provides a method of using the agent for projecting farm plants from such diseases.

**Claims**

1.   An agent for preventing and controlling stem and leaf diseases of plants, which contains cells belonging to the genus *Talaromyces* and antagonistic to fungi that cause stem and leaf diseases of plants.

2.   An agent for preventing and controlling stem and leaf diseases of plants, which comprises from 1 to 90 % by mass of cells belonging to the genus *Talaromyces* and antagonistic to fungi that cause stem and leaf diseases of plants, from 1 to 15 % by mass of a surfactant, and from 0 to 98 % by mass (including 0) of an extender.

3. An agent for preventing and controlling stem and leaf diseases of plants, which comprises from 2 to 46 % by mass of cells belonging to the genus *Talaromyces* and antagonistic to fungi that cause stem and leaf diseases of plants, from 2 to 15 % by mass of a surfactant, and from 39 to 96 % by mass of an extender.

4. The agent for preventing and controlling stem and leaf diseases of plants as claimed in any of claims 1 to 3, wherein the cells belonging to the genus *Talaromyces* are those of *Talaromyces flavus*.

5. The agent for preventing and controlling stem and leaf diseases of plants as claimed in any of claims 1 to 4, wherein the cells belonging to the genus *Talaromyces* are those of *Talaromyces flavus* Y-9401.

6. The agent for preventing and controlling stem and leaf diseases of plants as claimed in any of claims 1 to 5, wherein the cells belonging to the genus *Talaromyces* are cultured cells of from $1 \times 10^6$ to $1 \times 10^{12}$/g in terms of the colony-forming units, or ground powder or spores thereof.

7. The agent for preventing and controlling stem and leaf diseases of plants as claimed in claim 2 or 3, wherein the surfactant is one or more selected from fatty acid soaps, alkylether-carboxylic acids, N-acylamino acids, salts of alkylbenzenesulfonic acids, salts of alkylnaphthalenesulfonic acids, salts of $\alpha$-olefinsulfonic acids, salts of dialkylsulfosuccinate esters, salts of higher alcohol sulfate esters, salts of alkylether-sulfuric acids, salts of polyoxyethylene-alkylphenylether-sulfuric acids, salts of fatty acid alkylolamide sulfate esters, salts of alkylether phosphate esters, salts of alkyl phosphate esters, salts of aliphatic amines, aliphatic quaternary ammonium salts, benzalkonium salts, benzetonium chloride, pyridinium salts, imidazolinium salts, polyoxyethylene alkyl ethers, polyoxyethylene alkylphenyl ethers, polyoxyethylene-polyoxypropylene block polymers, polyoxyethylene-polyoxypropylene alkyl ethers, polyoxyethylene glycerin fatty acid esters, polyoxyethylene castor oil, polyoxyethylene sorbitan fatty acid esters, polyoxyethylene sorbitol fatty acid esters, polyethylene glycol fatty acid esters, fatty acid monoglycerides, polyglycerin fatty acid esters, sorbitan fatty acid esters, fatty acid alkanolamides, polyoxyethylene fatty acid amides, polyoxyethylene-alkylamines, alkylamine oxides, carboxybetaines, salts of aminocarboxylic acids, and imidazolinium betaines.

8. The agent for preventing and controlling stem and leaf diseases of plants as claimed in any of claim 2 or 3, wherein the extender is one or more selected from kaolin clay, pyrophyllite clay, bentonite, montmorillonite, diatomaceous earth, synthetic silicon oxide hydrate, acid clay, talc, clay, ceramic, quartz, sericite, vermiculite, pearlite, ohya stone, anthrax, limestone, coal ash, zeolite and attapulgite.

9. A method for preventing and controlling stem and leaf diseases of plants, which comprises spraying the stems and leaves of plants with an agent for preventing and controlling stem and leaf diseases of plants and in which the agent contains cells belonging to the genus *Talaromyces* and antagonistic to fungi that cause stem and leaf diseases of plants.

10. A method for preventing and controlling stem and leaf diseases of plants, which comprises spraying the stems and leaves of plants with a 50-fold to 3000-fold wet dilution of an agent for preventing and controlling stem and leaf diseases of plants and in which the agent comprises from 1 to 90 % by mass of cells belonging to the genus *Talaromyces* and antagonistic to fungi that cause stem and leaf diseases of plants, from 1 to 15 % by mass of a surfactant, and from 0 to 98 % by mass of an extender.

11. A method for preventing and controlling stem and leaf diseases of plants, which comprises spraying the stems and leaves of plants with a 50-fold to 3000-fold wet dilution of an agent for preventing and controlling stem and leaf diseases of plants and in which the agent comprises from 2 to 46 % by mass of cells belonging to the genus *Talaromyces* and antagonistic to fungi that cause stem and leaf diseases of plants, from 2 to 15 % by mass of a surfactant, and from 39 to 96 % by mass of an extender.

12. The method for preventing and controlling stem and leaf diseases of plants as claimed in any of claims 9 to 11, wherein the cells belonging to the genus *Talaromyces* are those of *Talaromyces flavus*.

13. The method for preventing and controlling stem and leaf diseases of plants as claimed in any of claims 9 to 12, wherein the cells belonging to the genus *Talaromyces* are those of *Talaromyces flavus* Y-9401.

14. The method for preventing and controlling stem and leaf diseases of plants as claimed in any of claims 9 to 13, wherein the cells belonging to the genus *Talaromyces* are cultured cells of from $1 \times 10^6$ to $1 \times 10^{12}$/g in terms of the colony-forming units, or ground powder or spores thereof.

15. The method for preventing and controlling stem and leaf diseases of plants as claimed in claim 10 or 11, wherein the surfactant is one or more selected from fatty acid soaps, alkylether-carboxylic acids, N-acylamino acids, salts of alkylbenzenesulfonic acids, salts of alkylnaphthalenesulfonic acids, salts of $\alpha$-olefinsulfonic acids, salts of dialkylsulfosuccinate esters, salts of higher alcohol sulfate esters, salts of alkylether-sulfuric acids, salts of polyoxyethylene-alkylphenylether-sulfuric acids, salts of fatty acid alkylolamide sulfate esters, salts of alkylether phosphate esters, salts of alkyl phosphate esters, salts of aliphatic amines, aliphatic quaternary ammonium salts, benzalkonium salts, benzetonium chloride, pyridinium salts, imidazolinium salts, polyoxyethylene alkyl ethers, polyoxyethylene alkylphenyl ethers, polyoxyethylene-polyoxypropylene block polymers, polyoxyethylene-polyoxypropylene alkyl ethers, polyoxyethylene glycerin fatty acid esters, polyoxyethylene castor oil, polyoxyethylene sorbitan fatty acid esters, polyoxyethylene sorbitol fatty acid esters, polyethylene glycol fatty acid esters, fatty acid monoglycerides, polyglycerin fatty acid esters, sorbitan fatty acid esters, fatty acid alkanolamides, polyoxyethylene fatty acid amides, polyoxyethylene-alkylamines, alkylamine oxides, carboxybetaines, salts of aminocarboxylic acids, and imidazolinium betaines.

16. The method for preventing and controlling stem and leaf diseases of plants as claimed in any of claim 10 or 11, wherein the extender is one or more selected from kaolin clay, pyrophyllite clay, bentonite, montmorillonite, diatomaceous earth, synthetic silicon oxide hydrate, acid clay, talc, clay, ceramic, quartz, sericite, vermiculite, pearlite, Ohya stone, anthrax, limestone, coal ash, zeolite and attapulgite.

17. The agent for preventing and controlling stem and leaf diseases of plants as claimed in any of claims 1 to 8, which is for gray mold, leaf blight or powdery mildew.

18. The method for preventing and controlling stem and leaf diseases of plants as claimed in any of claims 9 to 16, which is for gray mold, leaf blight or powdery mildew.

<div align="center">

**INTERNATIONAL SEARCH REPORT**

</div>

| | International application No. |
|---|---|
| | PCT/JP01/09394 |

| A. CLASSIFICATION OF SUBJECT MATTER |
|---|
| Int.Cl⁷ A01N63/04, C12N1/14 |
| |
| According to International Patent Classification (IPC) or to both national classification and IPC |

| B. FIELDS SEARCHED |
|---|
| Minimum documentation searched (classification system followed by classification symbols) |
| Int.Cl⁷ A01N63/04, A01N63/02, C12N1/14 |
| |
| Documentation searched other than minimum documentation to the extent that such documents are included in the fields searched |
| |
| Electronic data base consulted during the international search (name of data base and, where practicable, search terms used) |
| |

| C. DOCUMENTS CONSIDERED TO BE RELEVANT |
|---|

| Category* | Citation of document, with indication, where appropriate, of the relevant passages | Relevant to claim No. |
|---|---|---|
| X | JP 10-229872 A (Tochigi Pref. Gov.), 02 September, 1998 (02.09.1998), esp., Claims 1~4; Par. Nos. [0009],[0023] (Family: none) | 1-18 |
| X | JP 10-262459 A (Idemitsu Kosan Co., Ltd.), 06 October, 1998 (06.10.1998), esp., Claims 1, 5; Par. Nos. [0010],[0013]~[0014],[0017] (Family: none) | 1-18 |
| X | JP 10-262460 A (Idemitsu Kosan Co., Ltd.), 06 October, 1998 (06.10.1998), esp., Claims 1~5; Par. Nos. [0010]~[0011] (Family: none) | 1-18 |
| X | JP 2000-236871 A (Idemitsu Kosan Co., Ltd.), 05 September, 2000 (05.09.2000), esp., Claims 2~4; Par. Nos.[0002],[0012] (Family: none) | 1-18 |

| ☒ Further documents are listed in the continuation of Box C. | ☐ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 16 January, 2002 (16.01.02) | 29 January, 2002 (29.01.02) |

| Name and mailing address of the ISA/ | Authorized officer |
|---|---|
| Japanese Patent Office | |
| Facsimile No. | Telephone No. |

Form PCT/ISA/210 (second sheet) (July 1992)

## INTERNATIONAL SEARCH REPORT

| International application No. |
|---|
| PCT/JP01/09394 |

C (Continuation). DOCUMENTS CONSIDERED TO BE RELEVANT

| Category* | Citation of document, with indication, where appropriate, of the relevant passages | Relevant to claim No. |
|---|---|---|
| X | US 5068105 A (W. R. Grace & Co.-Conn.), 26 November, 1991 (26.11.1991), esp., Claims; Column 3, line 37; Column 4, lines 58~61 & EP 387640 A1 & JP 3-95105 A | 1-18 |
| A | JP 10-109913 A (Idemitsu Kosan Co., Ltd.), 28 April, 1998 (28.04.1998) (Family: none) | 1-18 |
| A | JP 11-302110 A (Mitsui Chemicals, Ltd.), 02 November, 1999 (02.11.1999) (Family: none) | 1-18 |

Form PCT/ISA/210 (continuation of second sheet) (July 1992)